(19) Europäisches Patentamt
European Patent Office
Office européen des brevets

(11) Veröffentlichungsnummer : **0 007 541**
**B1**

# (12) EUROPÄISCHE PATENTSCHRIFT

(45) Veröffentlichungstag der Patentschrift : **09.09.81**

(51) Int. Cl.³ : **C 07 D239/62**, A 01 N 43/54

(21) Anmeldenummer : **79102448.2**

(22) Anmeldetag : **16.07.79**

(54) **Substituierte 5-Phenylcarbamoyl-barbitursäuren, Verfahren zu ihrer Herstellung und ihre Verwendung als Insektizide.**

(30) Priorität : **26.07.78 DE 2832698**

(43) Veröffentlichungstag der Anmeldung : **06.02.80 (Patentblatt 80/03)**

(45) Bekanntmachung des Hinweises auf die Patenterteilung : **09.09.81 Patentblatt 81/36**

(84) Benannte Vertragsstaaten : **BE CH DE FR GB IT NL**

(56) Entgegenhaltungen : **FR - A - 2 350 344**

(73) Patentinhaber : **BAYER AG**
**Zentralbereich Patente, Marken und Lizenzen**
**D-5090 Leverkusen 1, Bayerwerk (DE)**

(72) Erfinder : **Krämer, Wolfgang, Dr.**
**Am Eckbusch 39/162**
**D-5600 Wuppertal 1 (DE)**
Erfinder : **Klauke, Erich, Dr.**
**Eichendorffweg 8**
**D-5068 Odenthal (DE)**
Erfinder : **Roessler, Peter, Dr.**
**Elster Strasse 15**
**D-5060 Bergisch-Gladbach 1 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 007 541 B1

Imprimerie Jouve, 17, rue du Louvre, 75001 Paris, France

Substituierte 5-Phenylcarbamoyl-barbitursäuren, Verfahren zu ihrer Herstellung und ihre Verwendung als Insektizide

Die vorliegende Erfindung betrifft neue substituierte 5-Phenylcarbamoyl-barbitursäuren, ein Verfahren zu ihrer Herstellung sowie ihre Verwendung als Insektizide, insbesondere als Inhibitoren der Entwicklung von Insekten.

Es ist bereits bekannt geworden, daß bestimmte 1,3-Di-alkyl-5-alkyl-bzw.-phenyl-carbamoyl-barbitursäure-Derivate zur Bekämpfung von Insekten eingesetzt werden können (vergleiche die japanische Patentschrift J5 1 123-823 ≙ Derwent 93 363 × /50 und die belgische Patentschrift 854 287). Deren Wirkung ist jedoch, insbesondere beim Einsatz als Inhibitoren der Entwicklung von Insekten, nicht immer ganz befriedigend.

Es wurden neue substituierte 5-Phenylcarbamoyl-barbitursäuren der allgemeinen Formel (I)

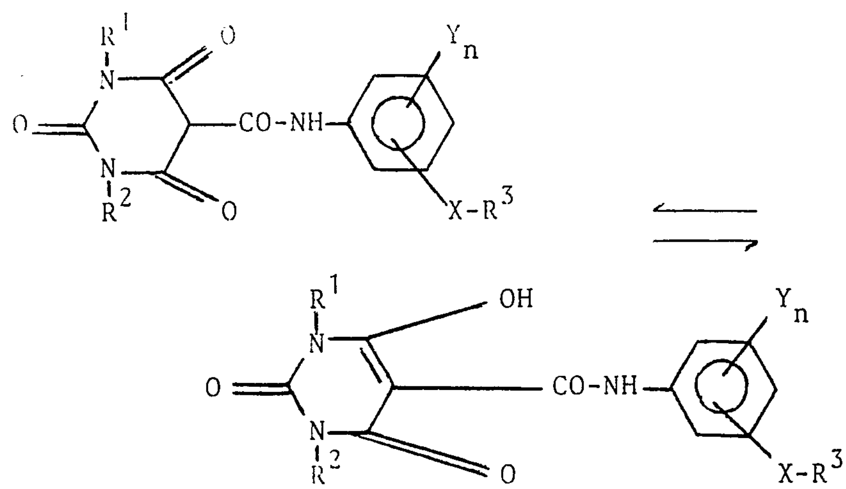

(I)

in welcher

$R^1$ für Wasserstoff oder Alkyl steht,

$R^2$ für Wasserstoff oder Alkyl steht,

$R^3$ für Halogenalkyl oder durch Halogenalkyl substituiertes Phenyl steht,

X für Sauerstoff oder Schwefel steht,

Y für Halogen oder Halogenalkyl steht,

n für 0, 1 oder 2 steht,

und X, $R^3$ und Y in ortho-Stellung zueinander gemeinsam auch für die Gruppe $-O-CF_2-O-CF_2-$stehen,

und deren Salze mit physiologisch verträglichen Basen gefunden. Sie weisen starke die Entwicklung von Insekten inhibierende Wirkungen auf.

Man erhält die substituierten 5-Phenylcarbamoyl-barbitursäuren der Formel (I), wenn man Barbitursäuren der Formel (II)

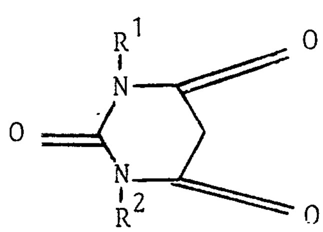

(II)

in welcher

$R^1$ und $R^2$ die oben angegebene Bedeutung haben, mit Isocyanaten der Formel (III)

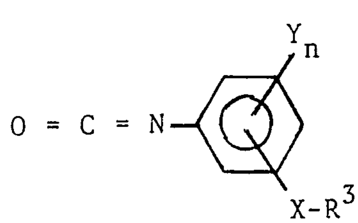

(III)

in welcher

$R^3$, X, Y und n die oben angegebene Bedeutung haben,

in Gegenwart eines Katalysators und gegebenenfalls in Gegenwart eines Verdünnungsmittels umsetzt.

Weiterhin können die erfindungsgemäß erhältlichen substituierten 5-Phenylcarbamoyl-barbitursäuren der Formel (I) durch Umsetzung mit Basen in die Salze überführt werden.

Überraschenderweise zeigen die erfindungsgemäßen substituierten 5-Phenylcarbamoyl-barbitursäuren eine höhere die Entwicklung von Insekten inhibierende Wirkung als die aus dem Stand der Technik bekannten 1,3-Dialkyl-5-alkyl-bzw.-phenyl-carbamoyl-barbitursäure-Derivate, welche die chemisch und wirkungsmäßig naheliegendsten Verbindungen sind. Die erfindungsgemäßen Wirkstoffe stellen somit eine Bereicherung der Technik dar.

Die erfindungsgemäßen substituierten 5-Phenylcarbamoyl-barbitursäuren sind durch die Formel (I) allgemein definiert. In dieser Formel stehen $R^1$ und $R^2$ vorzugsweise für Wasserstoff oder Alkyl mit 1 bis 4 Kohlenstoffatomen. $R^3$ steht vorzugsweise für Halogenalkyl mit bis zu 2 Kohlenstoff- und bis zu 5 gleichen oder verschiedenen Halogenatomen, wie insbesondere Fluor- und Chloratomen, sowie für Phenyl, das durch Halogenalkyl mit bis zu 2 Kohlenstoff- und bis zu 5 gleichen oder verschiedenen Halogenatomen, wie insbesondere Fluor- und Chloratomen, substituiert ist. *X* steht vorzugsweise für Sauerstoff und Schwefel. *Y* steht vorzugsweise für die Halogene Fluor, Chlor oder Brom sowie für Halogenalkyl mit bis zu 2 Kohlenstoff- und bis zu 5 gleichen oder verschiedenen Halogenatomen, wie insbesondere Fluor- und Chloratomen. Der Index *n* steht vorzugsweise für die Zahlen 0, 1 oder 2. Außerdem stehen der Rest $-X-R^3$ und *Y* in ortho-Stellung zueinander gemeinsam auch vorzugsweise für die Gruppe $-O-CF_2-O-CF_2-$.

Ganz besonders bevorzugt sind diejenigen Verbindungen der Formel (I), in denen $R^1$ und $R^2$ für Wasserstoff, Methyl, Ethyl oder Isopropyl stehen; $R^3$ für Trifluormethyl, 1,1,2-Trifluor-2-chlor-ethyl, 1,1,2,2-Tetrafluorethyl, 1,1,2,2-Tetrafluor-2-chlorethyl, Chlor-difluor-methyl, Dichlorfluormethyl oder Trichlormethyl sowie Trifluormethylphenyl steht; *X* für Sauerstoff oder Schwefel steht; *Y* für Fluor oder Chlor sowie Trifluormethyl steht; der Index *n* für die Zahlen 0, 1 oder 2 steht; und der Rest $-X-R^3$ mit *Y* in ortho-Stellung zueinander gemeinsam für die Gruppierung $-O-CF_2-O-CF_2-$stehen.

Im einzelnen seien außer den bei den Herstellungsbeispielen genannten Verbindungen die Folgenden Verbindungen der allgemeinen Formel (I) genannt:

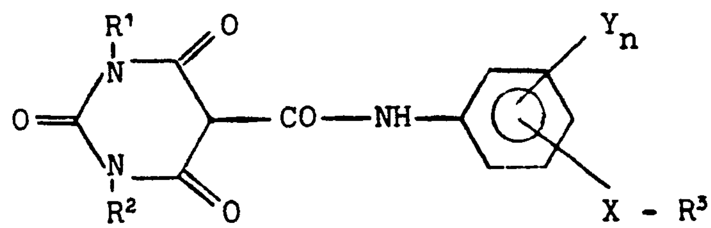

| $R^1$ | $R^2$ | $-X-R^3$ | $Y_n$ |
|---|---|---|---|
| $CH_3$ | $C_2H_5$ | $4-O-CF_3$ | 3-Cl |
| $CH_3$ | $C_2H_5$ | $4-S-CF_3$ | 3-Cl |
| $CH_3$ | $CH_3$ | $4-S-CF_3$ | 3-Cl |
| $C_2H_5$ | $C_2H_5$ | $4-S-CF_3$ | 3-Cl |
| $CH_3$ | $C_2H_5$ | $4-OCF_3$ | 2-Cl |
| $CH_3$ | $CH_3$ | $4-OCF_3$ | 2-Cl |
| $C_2H_5$ | $C_2H_5$ | $4-OCF_3$ | 2-Cl |
| $CH_3$ | $C_2H_5$ | $4-O-CF_2-CHClF$ | 3-Cl |
| $CH_3$ | $CH_3$ | $4-O-CF_2-CHClF$ | 3-Cl |
| $C_2H_5$ | $C_2H_5$ | $4-O-CF_2-CHClF$ | 3-Cl |
| $CH_3$ | $C_2H_5$ | $4-O-CF_3$ | — |
| $CH_3$ | $CH_3$ | $4-O-CF_3$ | — |
| $C_2H_5$ | $C_2H_5$ | $4-O-CF_3$ | — |
| $CH_3$ | $C_2H_5$ | $4-O-CF_2-CHF_2$ | — |
| $CH_3$ | $CH_3$ | $4-O-CF_2-CHF_2$ | — |
| $C_2H_5$ | $C_2H_5$ | $4-O-CF_2-CHF_2$ | — |
| $CH_3$ | $C_2H_5$ | $3-O-CF_2-CHClF$ | — |
| $CH_3$ | $CH_3$ | $3-O-CF_2-CHClF$ | — |
| $C_2H_5$ | $C_2H_5$ | $3-O-CF_2-CHClF$ | — |
| $CH_3$ | $C_2H_5$ | $4-O-CF_2-CF_2Cl$ | 3-Cl |
| $CH_3$ | $CH_3$ | $4-O-CF_2-CF_2Cl$ | 3-Cl |
| $C_2H_5$ | $C_2H_5$ | $4-O-CF_2-CF_2Cl$ | 3-Cl |
| $CH_3$ | $C_2H_5$ | $4-S-CF_2Cl$ | 3-Cl |
| $CH_3$ | $CH_3$ | $4-S-CF_2Cl$ | 3-Cl |

| $R^1$ | $R^2$ | $-X-R^3$ | $Y_n$ |
|---|---|---|---|
| $C_2H_5$ | $C_2H_5$ | $4-S-CH_2Cl$ | 3-Cl |
| $CH_3$ | $C_2H_5$ | $4-S-CF_3$ | — |
| $CH_3$ | $CH_3$ | $4-S-CF_3$ | — |
| $C_2H_5$ | $C_2H_5$ | $4-S-CF_3$ | — |
| $CH_3$ | $C_2H_5$ | 4-O- 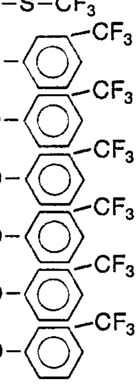 | — |
| $CH_3$ | $CH_3$ | 4-O- | — |
| $C_2H_5$ | $C_2H_5$ | 4-O- | — |
| $CH_3$ | $C_2H_5$ | 4-O- | $3-CF_3$ |
| $CH_3$ | $CH_3$ | 4-O- | $3-CF_3$ |
| $C_2H_5$ | $C_2H_5$ | 4-O- | $3-CF_3$ |
| $CH_3$ | $C_2H_5$ | $-O-CF_2-O-CF_2-$ | |
| $CH_3$ | $CH_3$ | $-O-CF_2-O-CF_2-$ | |
| $C_2H_5$ | $C_2H_5$ | $-O-CF_2-O-CF_2-$ | |

Verwendet man beispielsweise 1,2-Dimethyl-barbitursäure und 3-Chlor-4-trifluormethoxy-phenylisocyanat als Ausgangsstoffe, so kann der Reaktionablauf durch das folgende Formelschema wiedergegeben werden:

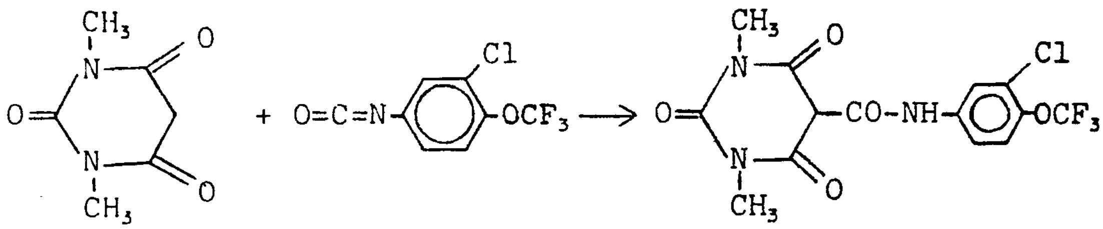

Die für das erfindungsgemäße Verfahren als Ausgangsstoffe zu verwendenden Barbitursäuren sind durch die Formel (II) allgemein definiert. In dieser Formel stehen $R^1$ und $R^2$ vorzugsweise für die Reste, die bei der Beschreibung der Verbindungen der Formel (I) bereits vorzugsweise genannt wurden.

Die Barbitursäuren der Formel (II) sind allgemein bekannte Verbindungen der organischen Chemie. Als Beispiele seien genannt:

1,3-Dimethyl-barbitursäure, 1-Methyl-barbitursäure, 1-Ethyl-3-methyl-barbitursäure, 1,3-Diethylbarbitursäure, 1-Ethylbarbitursäure, 1-Methyl-3-isopropyl-barbitursäure, 1-Methyl-3-propyl-barbitursäure, 1-Methyl-3-butyl-barbitursäure, 1-Methyl-3-isobutyl-barbitursäure, 1-Methyl-3-sek.-butyl-barbitursäure, 1-Methyl-3-tert.-butyl-barbitursäure, Barbitursäure.

Die außerdem für das erfindungsgemäße Verfahren als Ausgangsstoffe zu verwendenden Isocyanate sind durch die Formel (III) allgemein definiert. In dieser Formel stehen $R^3$, *X*, *Y* und *n* vorzugsweise für die Reste, die bei der Beschreibung der Verbindungen der Formel (I) bereits vorzugsweise genannt wurden.

Die Isocyanate der Formel (III) sind bekannt oder lassen sich nach allgemein üblichen und bekannten Verfahren herstellen, z.B. durch Umsetzung von Aminen mit Phosgen und anschließendem Erhitzen. Diese Verfahren sind aus den allgemeinen Lehrbüchern der organischen Chemie bekannt. Als Beispiele seien genannt:

4-Trifluormethoxyphenyl-isocyanat
4-Trifluormethylthiophenyl-isocyanat
2-Chlor-4-trifluormethoxyphenyl-isocyanat
3-Chlor-4-trifluormethoxyphenyl-isocyanat
2-Chlor-4-trifluormethylthiophenyl-isocyanat
3-Chlor-4-trifluormethylthiophenyl-isocyanat
4-Chlordifluormethoxyphenyl-isocyanat
4-Chlordifluormethylthiophenyl-isocyanat
2-Chlor-4-chlordifluormethoxyphenyl-isocyanat
3-Chlor-4-chlordifluormethoxyphenyl-isocyanat
2-Chlor-4-chlordifluormethylthiophenyl-isocyanat

3-Chlor-4-chlordifluormethylthiophenyl-isocyanat
2-Chlor-4-chlordifluormethylthiophenyl-isocyanat
3-Chlor-4-chlordifluormethylthiophenyl-isocyanat
4-(1,1,2,2-Tetrafluorethoxy)-phenyl-isocyanat
3-Chlor-4-(1,1,2,2-tetrafluorethoxy)-phenyl-isocyanat
2-Chlor-4-(1,1,2,2-tetrafluorethoxy)-phenyl-isocyanat
4-(1,1,2-Trifluor-2-chlor-ethoxy)-phenyl-(thio)-isocyanat
3-Chlor-4-(1,1,2-trifluor-2-chlor-ethoxy)-phenyl-isocyanat
3-(1,1,2-Trifluor-2-chlor-ethoxy)-phenyl-isocyanat
4-(1,1,2,2-Tetrafluor-2-chlor-ethoxy)-phenyl-isocyanat
3-Chlor-4-(1,1,2,2-tetrafluor-2-chlor-ethoxy)-phenyl-isocyanat
2-Chlor-4-(1,1,2,2-tetrafluor-2-chlor-ethoxy)-phenyl-isocyanat
4-(3'-Trifluormethylphenoxy)-phenyl-isocyanat
3-Trifluormethyl-4-(3'-trifluormethylphenoxy)-phenyl-isocyanat
2,2,4,4-Tetrafluor-1,3-benzodioxan-6-yl-isocyanat.

Zur Herstellung von Salzen der Verbindungen der Formel (I) kommen alle physiologisch verträglichen Basen infrage. Vorzugsweise seien Natrium-Salze, Kalium-Salze sowie insbesondere Ammonium-Salze und Trialkylamin-Salze, wie z.B. Triethylamin-Salze, genannt.

Als Verdünnungsmittel kommen bei der Durchführung des erfindungsgemäßen Verfahrens alle inerten organischen Lösungsmittel infrage. Hierzu gehören vorzugsweise Ether, wie Diethylether, Tetrahydrofuran oder Dioxan ; Ketone, wie Aceton und Methylethylketon ; aliphatische und aromatische Kohlenwasserstoffe, wie Ligroin, Petrolether, Benzol, Toluol oder Xylol ; sowie chlorierte Kohlenwasserstoffe, wie Tetrachlorkohlenstoff, Chloroform und Methylenchlorid.

Als Katalysatoren können bei der Durchführung des erfindungsgemäßen Verfahrens alle üblicherweise verwendbaren basischen Katalysatoren eingesetzt werden. Hierzu gehören vorzugsweise organische Basen, wie Triethylamin, Pyridin, und Zinn-organische Verbindungen, wie Dibutylzinndilaurat.

Die Reaktionstemperaturen können bei der Durchführung des erfindungsgemäßen Verfahrens in einem größeren Bereich variiert werden. Im allgemeinen arbeitet man zwischen 0 und 150 °C, vorzugsweise zwischen 20 und 100 °C.

Bei der Durchführung des erfindungsgemäßen Verfahrens setzt man auf 1 Mol Barbitursäure der Formel (II) vorzugsweise 1 Mol Isocyanat der Formel (III) und 0,1-1 Mol Katalysator ein. Zur Isolierung der Verbindungen der Formel (I) wird das Reaktionsgemisch angesäuert, im Vakuum eingeengt, der Rückstand mit Wasser versetzt ; abfiltriert, gut mit Alkohol gewaschen und getrocknet.

Die erfindungsgemäßen Wirkstoffe zeigen bei höheren Aufwandkonzentrationen fungizide Wirkungen.

Die Wirkstoffe eignen sich bei guter Pflanzenverträglichkeit und günstiger Warmblütertoxizität zur Bekämpfung von tierischen Schädlingen, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören :

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp.

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips fermoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Mephotettix cincticeps, Lecanium corni, Saissetia oleae, Laodelphax striatellus, Nilaparvata lugens, Aonidiella aurantii, Aspidiotus hederae, Pseudococcus spp., Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria,

Euproctis chrysorrhoea, Lymantria spp., Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp.

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage : Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser ; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid ; als feste Trägerstoffe kommen in Frage : z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate ; als feste Trägerstoffe für Granulate kommen in Frage : z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel ; als Emulgier- und/oder schaumerzeugende Mittel kommen in Frage : z.B. nichtionogene und anionische Emulgatoren, wie Polyoxyäthylen-Fettsäure-Ester, Polyoxyäthylen-Fettalkohol-Äther, z.B. Alkylarylpelyglykol-äther, Alkylsulfonate, Alkylsulfate, Arylsulfonate sowie Eiweißhydrolysate ; als Dispergiermittel kommen in Frage : z.B. Lignin-Sulfit ablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die Anwendung der erfindungsgemäßen Wirkstoffe erfolgt in Form ihrer handelsüblichen Formulierungen und/oder den aus diesen Formulierungen bereiteten Anwendungsformen.

Der Wirkstoffgehalt der aus den handelsüblichen Formulierungen bereiteten Anwendungsformen

kann in weiten Bereichen variieren. Die Wirkstoffkonzentration der Anwendungsformen kann von 0,0000001 bis zu 100 Gew.-% Wirkstoff, vorzugsweise zwischen 0,01 und 10 Gew.-%, liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Bei den folgenden Beispielen zur entwicklungshemmenden Wirkung der Wirkstoffe werden während der gesamten angegebenen Entwicklung der Testtiere die morphologischen Veränderungen, wie zur Hälfte verpuppte Tiere, unvollständig geschlüpfte Larven oder Raupen, defekte Flügel, pupale Kutikula bei Imagines etc., als Mißbildungen gewertet. Die Summe der Morphologischen Mißbildungen, zusammen mit den während des Häutungsgeschehens oder der Metamorphose abgetöteten Tieren, wird in Prozent der Gesamtzahl der eingesetzten Versuchstiere bestimmt.

## Beispiel A

Entwicklungshemmende Wirkung/Fraßtest

| | |
|---|---|
| Testtiere: | Plutella maculipennis (Raupen im 4. Entwicklungsstadium) |
| Zahl der Testtiere: | 20 Stück |
| Testtiere: | Phaedon cochleariae (Larven im 4. Entwicklungsstadium) |
| Zahl der Testtiere: | 20 Stück |
| Futterpflanzen: | Kohlpflanzen (Brassica olearacea) |
| Lösungsmittel: | 10 Gewichtsteile Aceton |
| Emulgator: | 1 Gewichtsteil Alkylarylpolyglykoläther |

Zur Herstellung einer zweckmäßigen Wirkstoffzubereitung vermischt man 2 Gewichtsteile Wirkstoff mit der angegebenen Menge Lösungsmittel, Emulgator und so viel Wasser, daß eine 1 %ige Mischung entsteht, die mit Wasser auf die gewünschte Konzentration verdünnt wird.

Die Testtiere werden mit Blättern der Futterpflanzen, die mit einem gleichmäßigen Spritzbelag der Wirkstoffzubereitung so überzogen werden, daß die gewünschte Wirkstoffkonzentration (Wirkstoffmenge pro Flächeneinheit) auf den Blättern erreicht wird, bis zur Entwicklung der Imago gefüttert.

Zur Kontrolle werden nur mit Lösungsmittel- und Emulgator-Wassergemisch der entsprechenden Konzentration überzogene Blätter verfüttert.

Bei diesem Test zeigen z.B. die folgenden Verbindungen der Herstellungsbeispiele überlegene Wirkung gegenüber dem Stand der Technik : 1, 2.

## Beispiel B

Entwicklungshemmende Wirkung/Laphygma-Raupen-Test

| | |
|---|---|
| Testtiere: | Laphygma frugiperda (Raupen) |
| Futter: | 1 cm dicke Scheibe von 3 cm Durchmesser luftangetrocknetes Kunstfutter aus Bohnenkernschrot, Hefe, Vitaminmischung, Blattpulver, Agar und Konservierungsstoff |
| Lösungsmittel: | 10 Gewichtsteile Aceton |
| Emulgator: | 1 Gewichtsteil Alkylarylpolyglykoläther |

Zur Herstellung einer zweckmäßigen Wirkstoffzubereitung vermischt man 2 Gewichtsteile Wirkstoff mit der angegebenen Menge Lösungsmittel, Emulgator und so viel Wasser, daß eine 1 %ige Mischung entsteht, die mit Wasser auf die gewünschte Konzentration verdünnt wird.

Jeweils ein Testtier wird auf eine mit 1,5 ml Wirkstoffzubereitung der gewünschten Konzentration angefeuchtete Futterscheibe gesetzt und bis zum Schlüpfen der Imago beobachtet.

Zur Kontrolle wird je ein Testtier auf eine mit 1,5 ml Lösungsmittel- und Emulgator-Wassergemisch der entsprechenden Konzentration angefeuchtete Futterscheibe gesetzt und bis zum Schlüpfen der Imago beobachtet.

Bei diesem Test zeigen z.B. die folgenden Verbindungen der Herstellungsbeispiele überlegende Wirkung gegenüber dem Stand der Technik : 1.

## Herstellungsbeispiele

### Beispiel 1

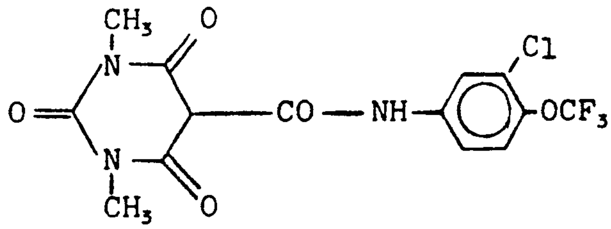

7,8 g (0,05 Mol) 1,3-Dimethylbarbitursäure werden in 150 ml Dioxan und 5 g (0,05 Mol) Triethylamin suspendiert und mit 11,85 g (0,05 Mol) 3-Chlor-4-trifluormethoxy-phenylisocyanat versetzt. Man läßt 5 Stunden unter Rückfluß rühren. Nach dem Abkühlen wird das Reaktionsgemisch mit 2n Salzsäure auf einen pH-Wert von 3 gebracht und im Vakuum durch Abdestillieren des Lösungsmittels eingeengt. Der Rückstand wird mit 200 ml Wasser versetzt, abgesaugt, mit 100 ml Isopropanol versetzt, erneut abgesaugt und getrocknet. Man erhält 16,5 g (74 % der Theorie) 5-(3'-Chlor-4'-trifluormethoxy-phenylcarbamoyl)-1,3-dimethyl-barbitursäure vom Schmelzpunkt 160 °C.

In analoger Weise werden die Verbindungen der Tabelle 1 erhalten.

Tabelle 1

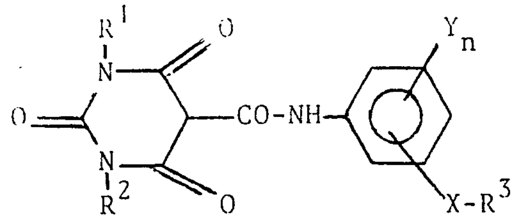

| Bsp. Nr. | $R^1$ | $R^2$ | $-XR^3$ | $Y_n$ | Schmelzpunkt (°C) |
|---|---|---|---|---|---|
| 2 | $C_2H_5$ | $C_2H_5$ | $4-OCF_3$ | 3−Cl | 134-35 |
| 3 | $C_2H_5$ | $CH_3$ | $4-OCF_3$ | 2−Cl | 119 |
| 4 | $C_2H_5$ | $CH_3$ | $4-OCF_2CHClF$ | 3−Cl | 124 |
| 5 | $C_2H_5$ | $CH_3$ | $4-OCF_2CF_2H$ | H | 119 |
| 6 | $C_2H_5$ | $CH_3$ | $4-OCF_3$ | H | 118-20 |
| 7 | $C_2H_5$ | $CH_3$ | $4-OCF_3$ | 3−Cl | 130-2 |
| 8 | $C_2H_5$ | $CH_3$ | $3-OCF_2CHClF$ | H | 98-99 |
| 9 | $C_2H_5$ | $CH_3$ | $4-SCF_2Cl$ | 3−Cl | 138-40 |
| 10 | $C_2H_5$ | $CH_3$ | $4-SCF_3$ | H | 142 |
| 11 | $C_2H_5$ | $CH_3$ | 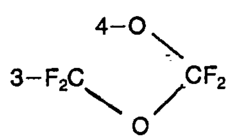 | | 98-100 |
| 12 | $C_2H_5$ | $CH_3$ | 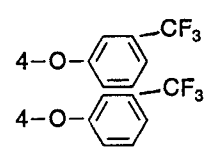 | H | 172-80 |
| 13 | $C_2H_5$ | $CH_3$ | | $3-CF_3$ | 63-70 |

**Ansprüche**

1. 5-Phenylcarbamoyl-barbitursäuren der allgemeinen Formel (I)

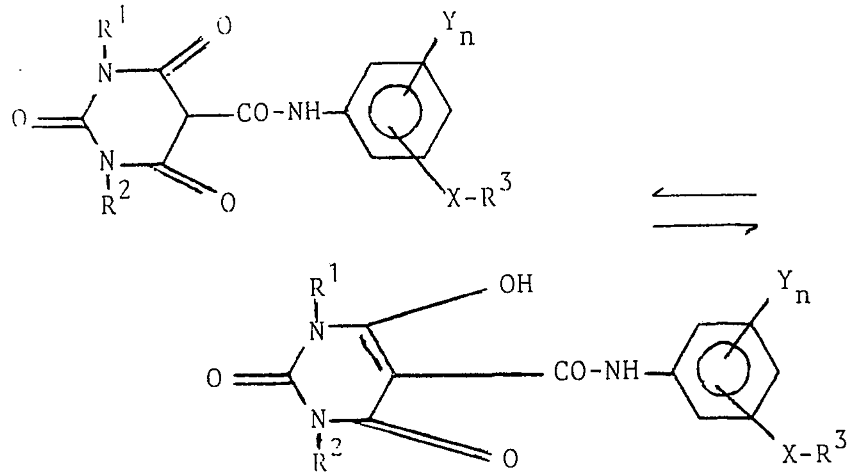

(I)

in welcher
$R^1$ für Wasserstoff oder Alkyl steht,
$R^2$ für Wasserstoff oder Alkyl steht,
$R^3$ für Halogenalkyl oder durch Halogenalkyl substituiertes Phenyl steht,
X für Sauerstoff oder Schwefel steht,
Y für Halogen oder Halogenalkyl steht,
n für 0, 1 oder 2 steht, und
X, $R^3$ und Y in ortho-Stellung zueinander gemeinsam auch für die Gruppe $-O-CF_2-O-CF_2-$stehen,
und deren Salze mit physiologisch verträglichen Basen.

2. Verfahren zur Herstellung der 5-Phenylcarbamoyl-barbitursäuren gemäß Anspruch 1, dadurch gekennzeichnet, daß man Barbitursäuren der Formel (II)

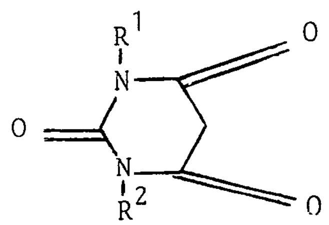

(II)

in welcher
$R^1$ und $R^2$ die oben angegebene Bedeutung haben, mit Isocyanaten der Formel (III)

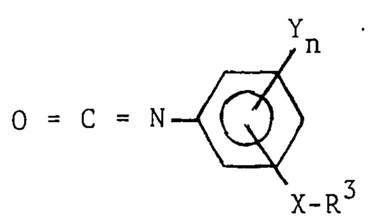

(III)

in welcher
$R^3$, X, Y und n die oben angegebene Bedeutung haben,
in Gegenwart eines Katalysators und gegebenenfalls in Gegenwart eines Verdünnungsmittels umsetzt.

3. Insektizide und akarizide Mittel, gekennzeichnet durch einen Gehalt an mindestens einer 5-Phenylcarbamoyl-barbitursäure der Formel (I).

4. Verwendung von 5-Phenylcarbamoyl-barbitursäuren der Formel (I) zur Bekämpfung von Insekten oder Spinnentieren.

5. Verfahren zur Herstellung insektizider und akarizider Mittel, dadurch gekennzeichnet, daß man 5-Phenylcarbamoyl-barbitursäuren der Formel (I) mit Streckmitteln und/oder oberflächenaktiven Mitteln vermischt.

**Claims**

1. 5-Phenylcarbamoyl-barbituric acids of the general formula (I)

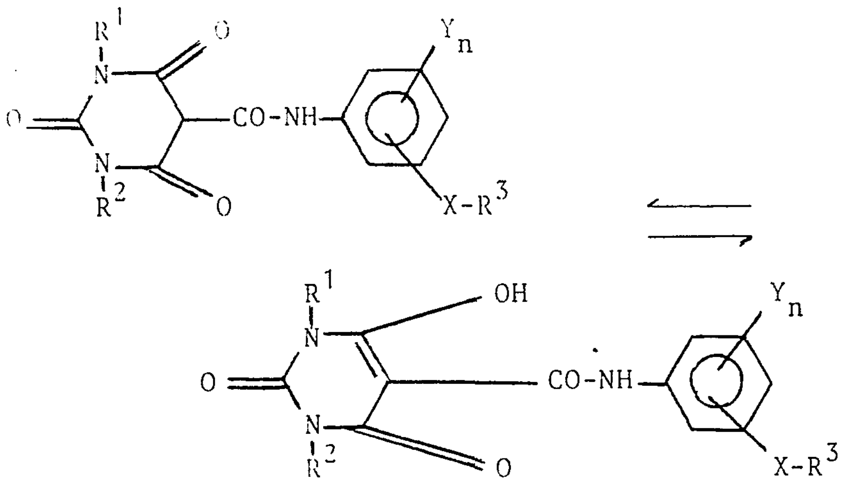

(I)

in which

$R^1$ represents hydrogen or alkyl,
$R^2$ represents hydrogen or alkyl,
$R^3$ represents halogenoalkyl, or phenyl substituted by halogenoalkyl,
X represents oxygen or sulphur,
Y represents halogen or halogenoalkyl,
n represents 0, 1 or 2 and
X, $R^3$ and Y in the ortho-position to one another conjointly also represent the $-O-CF_2-O-CF_2-$ group,
and their salts with physiologically tolerated bases.

2. Process for the preparation of the 5-phenylcarbamoyl-barbituric acids according to claim 1, characterised in that barbituric acids of the formula (II)

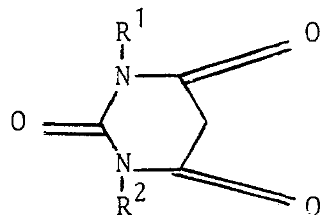

(II)

in which
$R^1$ and $R^2$ have the abovementioned meaning are reacted with isocyanates of the formula (III)

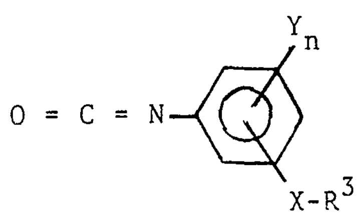

(III)

in which
$R^3$, X, Y and n have the abovementioned meaning,
in the presence of a catalyst and, if appropriate, in the presence of a diluent.

3. Insecticidal and acaricidal agents, characterised in that they contain at least one 5-phenylcarbamoyl-barbituric acid of the formula (I).

4. Use of 5-phenylcarbamoyl-barbituric acids of the formula (I) for combating insects or arachnidae.

5. Process for the preparation of insecticidal and acaricidal agents, characterised in that 5-phenylcarbamoyl-barbituric acids of the formula (I) are mixed with extenders and/or surface-active agents.

**Revendications**

1. Acides 5-phényl-carbamoyl-barbituriques de formule générale (I)

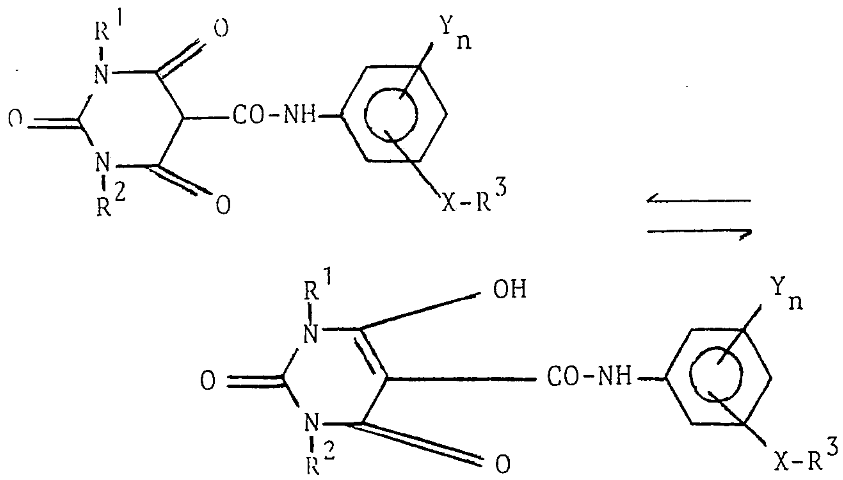

(I)

dans laquelle

$R^1$ représente un atome d'hydrogène ou un groupe alkyle,

$R^2$ représente un atome d'hydrogène ou un groupe alkyle,

$R^3$ représente un groupe halogénalkyle ou un groupe phényle substitué par un groupe halogénalkyle,

X représente un atome d'oxygène ou un atome de soufre,

Y représente un atome d'halogène ou un groupe halogénalkyle,

n représente 0, 1 ou 2, et

X, $R^3$ et Y, ensemble et en position ortho l'un par rapport à l'autre, représentent également le groupe $-O-CF_2-O-CF_2-$,

de même que les sels de ces acides avec des bases physiologiquement compatibles.

2. Procédé de préparation d'acides 5-phényl-carbamoyl-barbituriques suivant la revendication 1, caractérisé en ce qu'on fait réagir des acides barbituriques de formule (II)

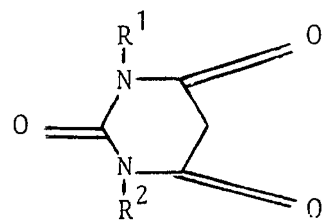

(II)

dans laquelle

$R^1$ et $R^2$ ont les significations indiquées ci-dessus, avec des isocyanates de formule (III)

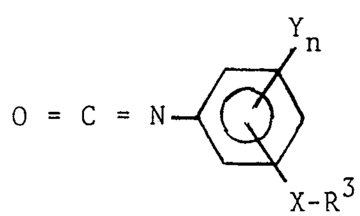

(III)

dans laquelle

$R^3$, X, Y et n ont les significations indiquées ci-dessus,

en présence d'un catalyseur et éventuellement en présence d'un diluant.

3. Agents insecticides et acaricides, caractérisés en ce qu'ils contiennent au moins un acide 5-phényl-carbamoyl-barbiturique de formule (I).

4. Utilisation d'acides 5-phényl-carbamoyl-barbituriques de formule (I) en vue de combattre les insectes ou les bombyx.

5. Procédé de préparation d'agents insecticides et acaricides, caractérisé en ce qu'on mélange des acides 5-phényl-carbamoyl-barbituriques de formule (I) avec des diluants et/ou des agents tensio-actifs.